# EUROPEAN PATENT APPLICATION

(11) **EP 3 748 006 A1**
(43) Date of publication of application: **09.12.2020**
(21) Application number: 20174958.7
(22) Date of filing: 15.05.2020
(51) Int. Cl.: C12N 15/82, A01H 5/00, B60C 1/00, C12N 9/10

(54) **METHOD FOR PRODUCING NATURAL RUBBER, TRANSGENIC PLANT, METHOD FOR PRODUCING PNEUMATIC TIRE, AND METHOD FOR PRODUCING RUBBER PRODUCT**

(30) Priority: 03.06.2019 JP 2019103713
(71) Applicant: SUMITOMO RUBBER INDUSTRIES, LTD., Chuo-ku Kobe-shi Hyogo 651-0072 (JP)
(72) Inventor: SAKURAI, Yuko, Chuo-ku, Kobe-shi, Hyogo 651-0072 (JP); INOUE, Yukino, Chuo-ku, Kobe-shi, Hyogo 651-0072 (JP); YAMAGUCHI, Haruhiko, Chuo-ku, Kobe-shi, Hyogo 651-0072 (JP); OKADA, Akari, Chuo-ku, Kobe-shi, Hyogo 651-0072 (JP)
(74) Representative: TBK

(57) **Abstract**

To provide methods for producing a natural rubber which enable the production of a natural rubber with an increased molecular weight, and transgenic plants which are able to produce a natural rubber with an increased molecular weight. Included is a method for producing a natural rubber, the method including the steps of: introducing a gene coding for a cis-prenyltransferase (CPT) family protein derived from a rubber-producing plant A into a rubber-producing plant B to prepare a transgenic plant C; and producing a natural rubber using the transgenic plant C, the rubber-producing plant A producing a natural rubber having a higher molecular weight than a natural rubber produced by the rubber-producing plant B not transfected with the gene.

## Description

### TECHNICAL FIELD

The present invention relates to methods for producing a natural rubber, transgenic plants, methods for producing a pneumatic tire, and methods for producing a rubber product.

### BACKGROUND ART

At present, natural rubbers used in industrial rubber products may be obtained by cultivating rubber-producing plants, such as para rubber trees (*Hevea brasiliensis*) belonging to the family *Euphorbiaceae* or Indian rubber trees (*Ficus elastica*) belonging to the family *Moraceae,* whose laticifer cells biosynthesize natural rubbers, and manually harvesting the natural rubbers from the plants.

Currently, *Hevea brasiliensis* is substantially the only source of natural rubber used in industrial rubber products. *Hevea brasiliensis* is a plant that can grow in limited regions, including Southeast Asia and South America. Moreover, *Hevea brasiliensis* trees take about seven years from planting to grow mature enough to yield rubber, and they yield the natural rubber only for a period of 20 to 30 years. Although the demand for natural rubbers is expected to increase in years to come, especially in developing countries, it is difficult for the above reasons to greatly increase natural rubber production from *Hevea brasiliensis.* Therefore, there has been a concern that natural rubber resources might dry up, and it has been desirable to provide stable natural rubber sources other than mature *Hevea brasiliensis* trees and to improve productivity of natural rubber from *Hevea brasiliensis.*

### SUMMARY OF INVENTION

### TECHNICAL PROBLEM

The present inventors made extensive studies and found that natural rubbers produced by rubber-producing plants other than *Hevea brasiliensis* have physical properties inferior to those of the natural rubber produced by *Hevea brasiliensis.* As a result of extensive research and experimentation to find the cause for this, the inventors discovered that the molecular weight of a producible natural rubber substantially depends on the type of rubber-producing plant, and that rubber-producing plants other than *Hevea brasiliensis* produce natural rubbers with lower molecular weights than the natural rubber produced by *Hevea brasiliensis,* and thus the natural rubbers produced by rubber-producing plants other than *Hevea brasiliensis* have physical properties inferior to those of the natural rubber produced by *Hevea brasiliensis.* In other words, as a result of the studies by the inventors, it was found that in order to use rubber-producing plants other than *Hevea brasiliensis* as stable natural rubber sources or to produce natural rubbers having better physical properties, it is necessary to increase the molecular weight of the natural rubbers produced by the rubber-producing plants.

The present invention aims to solve the problems discovered by the inventors and provide methods for producing a natural rubber which enable the production of a natural rubber with an increased molecular weight and transgenic plants which are able to produce a natural rubber with an increased molecular weight.

### SOLUTION TO PROBLEM

Natural rubbers have a cis-1,4-polyisoprene structure formed of isopentenyl diphosphate (IPP) as an elementary unit. This structure suggests that cis-prenyltransferase (CPT) may be involved in natural rubber biosynthesis.

The results of the extensive studies by the inventors show that an increase or decrease in CPT gene transcription caused no change in the molecular weight of the produced natural rubber. In contrast, it was discovered that when a CPT gene derived from a rubber-producing plant which produces a natural rubber with a high molecular weight is introduced into a rubber-producing plant which produces a natural rubber with a low molecular weight, the molecular weight of the natural rubber produced by the resulting transgenic plant is increased. Based on the discovery, the present invention has been accomplished.

Specifically, the present invention relates to a method for producing a natural rubber, the method including the steps of:
introducing a gene coding for a cis-prenyltransferase (CPT) family protein derived from a rubber-producing plant A into a rubber-producing plant B to prepare a transgenic plant C; and
producing a natural rubber using the transgenic plant C,
the rubber-producing plant A producing a natural rubber having a higher molecular weight than a natural rubber produced by the rubber-producing plant B not transfected with the gene. Hereinafter, this invention is described as the first aspect of the present invention and also referred to as the first invention.

The gene coding for a cis-prenyltransferase (CPT) family protein is preferably a gene coding for a cis-prenyltransferase (CPT) family protein derived from a plant of the genus *Hevea, Ficus, Taraxacum,* or *Parthenium.*

The gene coding for a cis-prenyltransferase (CPT) family protein is preferably a gene coding for a cis-prenyltransferase (CPT) family protein derived from *Hevea brasiliensis, Taraxacum kok-saghyz,* or *Parthenium argentatum.*

The present invention also relates to a method for producing a pneumatic tire, the method including the steps of:
kneading a natural rubber produced by the above-mentioned method for producing a natural rubber with an additive to obtain a kneaded mixture;
forming a green tire from the kneaded mixture; and
vulcanizing the green tire.

The present invention also relates to a method for producing a rubber product, the method including the steps of:
kneading a natural rubber produced by the above-mentioned method for producing a natural rubber with an additive to obtain a kneaded mixture;
forming a raw rubber product from the kneaded mixture; and
vulcanizing the raw rubber product.

The present invention also relates to a transgenic plant, produced by introducing a gene coding for a cis-prenyltransferase (CPT) family protein derived from a rubber-producing plant A into a rubber-producing plant B, the rubber-producing plant A producing a natural rubber having a higher molecular weight than a natural rubber produced by the rubber-producing plant B not transfected with the gene. Hereinafter, this invention is described as the second aspect of the present invention and also referred to as the second invention.

### ADVANTAGEOUS EFFECTS OF THE INVENTION

The first invention relates to a method for producing a natural rubber, the method including the steps of: introducing a gene coding for a cis-prenyltransferase (CPT) family protein derived from a rubber-producing plant A into a rubber-producing plant B to prepare a transgenic plant C; and producing a natural rubber using the transgenic plant C, the rubber-producing plant A producing a natural rubber having a higher molecular weight than a natural rubber produced by the rubber-producing plant B not transfected with the gene. This method thus enables the production of a natural rubber with an increased molecular weight.

The method for producing a pneumatic tire of the first invention includes the steps of: kneading a natural rubber produced by the method for producing a natural rubber of the first invention with an additive to obtain a kneaded mixture; forming a green tire from the kneaded mixture; and vulcanizing the green tire. This method uses a natural rubber with an increased molecular weight to produce a pneumatic tire, and thus enables the production of a pneumatic tire having good properties.

The method for producing a rubber product of the first invention includes the steps of: kneading a natural rubber produced by the method for producing a natural rubber of the first invention with an additive to obtain a kneaded mixture; forming a raw rubber product from the kneaded mixture; and vulcanizing the raw rubber product. This method uses a natural rubber with an increased molecular weight to produce a rubber product, and thus enables the production of a rubber product having good properties.

The transgenic plant of the second invention is produced by introducing a gene coding for a cis-prenyltransferase (CPT) family protein derived from a rubber-producing plant A into a rubber-producing plant B, the rubber-producing plant A producing a natural rubber having a higher molecular weight than a natural rubber produced by the rubber-producing plant B not transfected with the gene. It is thus possible to provide a transgenic plant which is able to produce a natural rubber with an increased molecular weight.

### BRIEF DESCRIPTION OF DRAWINGS

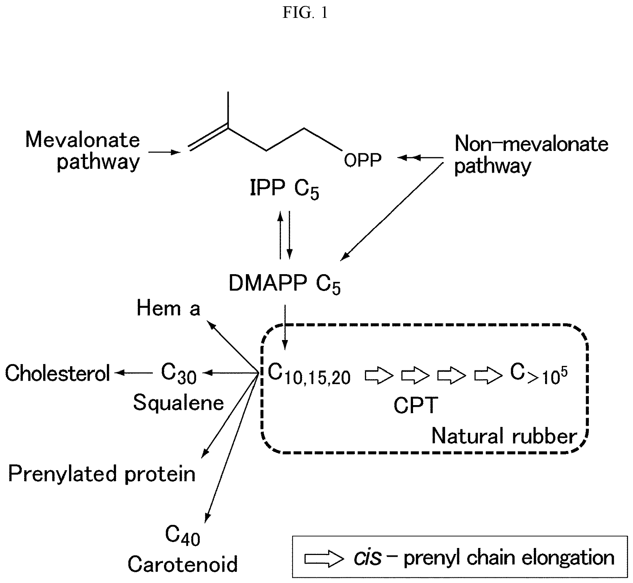

The accompanying figure schematically illustrates a part of the biosynthetic pathway of a natural rubber.

### DESCRIPTION OF EMBODIMENTS

Herein, the first and second inventions are also referred to collectively as the present invention. The first invention includes a method for producing a natural rubber, a method for producing a pneumatic tire, and a method for producing a rubber product, all of which use the transgenic plant of the second invention. Thus, it would be understood that the following description of the first invention encompasses the description of the second invention.

The method for producing a natural rubber of the first invention includes the steps of:
introducing a gene coding for a cis-prenyltransferase (CPT) family protein derived from a rubber-producing plant A into a rubber-producing plant B to prepare a transgenic plant C; and
producing a natural rubber using the transgenic plant C,
wherein the rubber-producing plant A produces a natural rubber having a higher molecular weight than the natural rubber produced by the rubber-producing plant B not transfected with the gene.

The transgenic plant of the second invention is produced by introducing a gene coding for a cis-prenyltransferase (CPT) family protein derived from a rubber-producing plant A into a rubber-producing plant B, wherein the rubber-producing plant A produces a natural rubber having a higher molecular weight than the natural rubber produced by the rubber-producing plant B not transfected with the gene.

Herein, the gene coding for a cis-prenyltransferase (CPT) family protein is also referred to as a CPT gene.

As described above, the present inventors discovered that an increase or decrease in CPT gene transcription causes no change in the molecular weight of the produced natural rubber and, in contrast, that when a CPT gene derived from a rubber-producing plant which produces a natural rubber with a high molecular weight is introduced into a rubber-producing plant which produces a natural rubber with a low molecular weight, the molecular weight of the natural rubber produced by the resulting transgenic plant is increased. In other words, it is highly possible that the molecular weight of the produced natural rubber depends on the nature, not the amount, of the CPT. It was thus discovered that a CPT derived from a rubber-producing plant which produces a natural rubber with a high molecular weight has a higher ability to elongate the isoprene chain than a CPT derived from a rubber-producing plant which produces a natural rubber with a low molecular weight.

For example, a natural rubber derived from *Hevea brasiliensis* has a weight average molecular weight of 6.8 × 10⁵ to 3.0 × 10⁶ (J. Rubb. Res., 2(3), 150-159, incorporated herein by reference), and a natural rubber derived from *Lactuca sativa* has a weight average molecular weight of 1.3 × 10⁶ (Phytochemistry 67(2006), 2590-2596, incorporated herein by reference). According to both documents, the natural rubber produced by *Hevea brasiliensis* has a bimodal molecular weight distribution (i.e., containing a high molecular weight component and a low molecular weight component), while the natural rubber produced by *Lactuca sativa* has a unimodal molecular weight distribution with only a low molecular weight component (peak top: 1.3 × 10⁶). It is clear that since the natural rubber produced by *Hevea brasiliensis* has a weight average molecular weight of 6.8 × 10⁵ to 3.0 × 10⁶ but has a bimodal (two peaks) molecular weight distribution, the component on the high molecular weight side apparently has a higher molecular weight than the natural rubber produced by *Lactuca sativa.* In other words, the natural rubber produced by *Hevea brasiliensis* has a higher molecular weight than the natural rubber produced by *Lactuca sativa.* Then, the inventors discovered that when a CPT gene derived from *Hevea brasiliensis* is introduced into *Lactuca sativa* to prepare a transgenic plant (transgenic *Lactuca sativa*)*,* the transgenic plant can be used to produce a natural rubber having a higher molecular weight than the natural rubber produced by the untransgenic *Lactuca sativa.* This is because the CPT derived from a rubber-producing plant which produces a natural rubber with a high molecular weight has a higher ability to elongate the isoprene chain than the CPT derived from a rubber-producing plant which produces a natural rubber with a low molecular weight.

As described, a natural rubber having an increased molecular weight can be produced by introducing a CPT gene derived from a rubber-producing plant A which produces a natural rubber having a high molecular weight into a rubber-producing plant B which produces a natural rubber having a lower molecular weight than that of the rubber-producing plant A to prepare a transgenic plant C, and producing a natural rubber using the transgenic plant C. Owing to the increase in molecular weight, the natural rubber having an increased molecular weight has good properties (for example, abrasion resistance, tensile strength, fracture strength, low heat-buildup properties) and can be used to produce rubber products (for example, pneumatic tires) having good properties.

Herein, by "the rubber-producing plant A producing a natural rubber having a higher molecular weight than the natural rubber produced by the rubber-producing plant B not transfected with the gene" it is not meant that the rubber-producing plant A produces a natural rubber having a higher weight average molecular weight than the natural rubber produced by the rubber-producing plant B not transfected with the gene, but that the rubber-producing plant A produces a natural rubber whose highest molecular weight component has a higher molecular weight than the highest molecular weight component of a natural rubber produced by the rubber-producing plant B not transfected with the gene.

Hence, to determine which of two rubber-producing plants corresponds to the rubber-producing plant A or rubber-producing plant B, one may compare the highest molecular weight components of the natural rubbers produced by the rubber-producing plants to determine the rubber-producing plant producing the component with a higher molecular weight as the rubber-producing plant A.

Non-limiting examples of rubber-producing plants that may be used in the present invention include: the genus *Hevea,* e.g. *Hevea brasiliensis;* the genus *Sonchus,* e.g. *Sonchus oleraceus, Sonchus asper,* and *Sonchus brachyotus*; the genus *Solidago,* e.g. *Solidago altissima, Solidago virgaurea* subsp. *asiatica, Solidago virgaurea* subsp. *leipcarpa, Solidago virgaurea* subsp. *leipcarpa* f. *paludosa, Solidago virgaurea* subsp. *gigantea,* and *Solidago gigantea* Ait. var. *leiophylla* Fernald; the genus *Helianthus,* e.g. *Helianthus annus, Helianthus argophyllus, Helianthus atrorubens, Helianthus debilis, Helianthus decapetalus,* and *Helianthus giganteus;* the genus *Taraxacum,* e.g. dandelion (Taraxacum), *Taraxacum venustum* H. Koidz, *Taraxacum hondoense* Nakai, *Taraxacum platycarpum* Dahlst, *Taraxacum japonicum, Taraxacum officinale* Weber, *Taraxacum kok-saghyz,* and *Taraxacum brevicorniculatum*; the genus *Ficus,* e.g. *Ficus carica, Ficus elastica, Ficus pumila* L., *Ficus erecta* Thumb., *Ficus ampelas* Burm. f., *Ficus benguetensis* Merr., *Ficus irisana* Elm., *Ficus microcarpa* L. f., *Ficus septica* Burm. f., and *Ficus benghalensis;* the genus *Parthenium,* e.g. *Parthenium argentatum, Parthenium hysterophorus,* and *Ambrosia artemisiifolia*; lettuce (*Lactuca sativa*); and *Arabidopsis thaliana.*

Then, the rubber-producing plants to be used as the rubber-producing plants A and B may be determined in view of the molecular weights of the natural rubbers produced by rubber-producing plants according to the above-mentioned technique.

The rubber-producing plant A may be any rubber-producing plant that produces a natural rubber having a high molecular weight. For example, it is preferably a plant of the genus *Hevea, Ficus, Taraxacum,* or *Parthenium,* more preferably *Hevea brasiliensis, Taraxacum kok-saghyz,* or *Parthenium argentatum.*

Among these, the rubber-producing plant A is preferably a plant of the genus *Hevea* or *Taraxacum,* more preferably *Hevea brasiliensis* or *Taraxacum kok-saghyz.*

The rubber-producing plant B may be any rubber-producing plant that produces a natural rubber having a lower molecular weight than that of the rubber-producing plant A. Examples include *Lactuca sativa.*

The rubber-producing plant B is also preferably a plant of the genus *Hevea* or *Taraxacum,* more preferably *Hevea brasiliensis* or *Taraxacum kok-saghyz* because the use of a rubber-producing plant which originally produces a natural rubber with a high molecular weight as the rubber-producing plant B enables the production of a natural rubber with a higher molecular weight.

In the case where the rubber-producing plant B is a plant of the genus *Taraxacum,* preferably *Taraxacum kok-saghyz,* the rubber-producing plant A may be *Hevea brasiliensis,* for example.

The usable cis-prenyltransferase (CPT) family protein may be any enzyme that catalyzes a reaction of cis-chain elongation of an isoprenoid compound. Specifically, in plants, for example, a natural rubber is biosynthesized via a natural rubber biosynthesis pathway as shown in the accompanying figure. In the pathway, the CPT family protein is considered to be an enzyme that catalyzes the reaction enclosed by the dotted frame in the figure. The CPT family protein is characterized by having an amino acid sequence contained in the cis-IPPS domain (NCBI accession No. cd00475).

Herein, the term "isoprenoid compound" refers to a compound containing an isoprene unit (C₅H₈). Also, the term "cis-isoprenoid" refers to a compound including an isoprenoid compound in which isoprene units are cis-bonded, and examples include cis-farnesyl diphosphate, undecaprenyl diphosphate, and natural rubber.

Examples of the CPT family protein include *Hevea brasiliensis*-derived CPTs (HRT1, HRT2, CPT3 to CPT5), *Lactuca* sativa-derived CPT1 to CPT3, *Taraxacum brevicorniculatum*-derived CPT1 to CPT3, and *Parthenium* argentatum-derived CPT1 to CPT4.

Specific examples of the CPT family protein include the following protein [1]:
[1] a protein having the amino acid sequence represented by SEQ ID NO:2.
   Moreover, it is known that proteins having one or more amino acid substitutions, deletions, insertions, or additions relative to the original amino acid sequence can still have the inherent function. Thus, specific examples of the CPT family protein also include the following protein [2]:
[2] a protein having an amino acid sequence containing one or more amino acid substitutions, deletions, insertions, and/or additions relative to the amino acid sequence represented by SEQ ID NO:2, and having an enzyme activity that catalyzes a reaction of cis-chain elongation of an isoprenoid compound.

In order to maintain the function of the CPT family protein, it preferably has an amino acid sequence containing one or more, more preferably 1 to 58, still more preferably 1 to 44, further preferably 1 to 29, particularly preferably 1 to 15, most preferably 1 to 6, yet most preferably 1 to 3 amino acid substitutions, deletions, insertions, and/or additions relative to the amino acid sequence of SEQ ID NO:2.

Among other amino acid substitutions, conservative substitutions are preferred. Specific examples include substitutions within each of the following groups in the parentheses: (glycine, alanine), (valine, isoleucine, leucine), (aspartic acid, glutamic acid), (asparagine, glutamine), (serine, threonine), (lysine, arginine), and (phenylalanine, tyrosine).

It is also known that proteins with amino acid sequences having high sequence identity to the original amino acid sequence can also have similar functions. Thus, specific examples of the CPT family protein also include the following protein [3]:
[3] a protein having an amino acid sequence with at least 80% sequence identity to the amino acid sequence represented by SEQ ID NO:2, and having an enzyme activity that catalyzes a reaction of cis-chain elongation of an isoprenoid compound.

In order to maintain the function of the CPT family protein, the sequence identity to the amino acid sequence of SEQ ID NO:2 is preferably at least 85%, more preferably at least 90%, still more preferably at least 95%, particularly preferably at least 98%, most preferably at least 99%.

Herein, the sequence identity between amino acid sequences or nucleotide sequences may be determined using the algorithm BLAST [Pro. Natl. Acad. Sci. USA, 90, 5873 (1993), incorporated herein by reference] developed by Karlin and Altschul or FASTA [Methods Enzymol., 183, 63 (1990), incorporated herein by reference].

Whether it is a protein having the above enzyme activity may be determined by conventional techniques, such as by expressing a target protein in a transformant produced by introducing a gene coding for the target protein into *Escherichia coli* or other host organisms, and determining the presence or absence of the function of the target protein by the corresponding activity measuring method.

The gene coding for a CPT family protein (CPT gene) may be any gene that codes for a CPT family protein to express and produce the CPT family protein.

The CPT gene may be any of the CPT genes coding for the known CPT family proteins listed above or may be a CPT gene coding for an unknown CPT family protein.

When it is desired to use a CPT gene coding for an unknown CPT family protein, screening may be performed by a known technique using, for example, a part of the nucleotide sequence of a known CPT gene as a probe to identify the DNA coding for the CPT family protein, followed by isolating the DNA. The method of isolating a DNA molecule of interest using a DNA molecule as a probe is described in, for example, Molecular Cloning, 2nd edition, Cold Spring Harbor Press (1989), incorporated herein by reference.

The known CPT gene used as a probe here may be a CPT gene coding for a CPT family protein derived from an organism other than rubber-producing plants, such as AtCPT1 to AtCPT9 from *Arabidopsis thaliana,* undecaprenyl pyrophosphate synthase (UPPS) from *Escherichia coli,* undecaprenyl diphosphate synthase (UPS) from *Micrococcus,* RER 2 from yeast, DDPS from tobacco, DDPS from mouse, or HDS from human.

The gene coding for a cis-prenyltransferase (CPT) family protein may be any CPT gene derived from a rubber-producing plant which produces a natural rubber with a high molecular weight. For example, it is preferably a CPT gene derived from a plant of the genus *Hevea, Ficus, Taraxacum,* or *Parthenium,* more preferably a CPT gene derived from *Hevea brasiliensis, Taraxacum kok-saghyz,* or *Parthenium argentatum.*

Among these, the CPT gene is preferably a CPT gene derived from a plant of the genus *Hevea* or *Taraxacum,* more preferably a CPT gene derived from *Hevea brasiliensis* or *Taraxacum kok-saghyz.*

Specific examples of the CPT gene include the following DNAs [1] and [2]:
[1] a DNA having the nucleotide sequence represented by SEQ ID NO:1; and
[2] a DNA which hybridizes under stringent conditions to a DNA having a nucleotide sequence complementary to the nucleotide sequence represented by SEQ ID NO:1, and which codes for a protein having an enzyme activity that catalyzes a reaction of cis-chain elongation of an isoprenoid compound.

Herein, the term "hybridize" means a process in which a DNA hybridizes to a DNA having a specific nucleotide sequence or a part of the DNA. Accordingly, the DNA having a specific nucleotide sequence or a part of the DNA may have a nucleotide sequence long enough to be usable as a probe in Northern or Southern blot analysis or as an oligonucleotide primer in polymerase chain reaction (PCR) analysis. The DNA used as a probe may have a length of at least 100 bases, preferably at least 200 bases, more preferably at least 500 bases, although it may be a DNA of at least 10 bases, preferably at least 15 bases.

Techniques to perform DNA hybridization experiments are well known. The hybridization conditions under which experiments are carried out may be determined in accordance with, for example, Molecular Cloning, 2nd ed. and 3rd ed. (2001), Methods for General and Molecular Bacteriology, ASM Press (1994), Immunology methods manual, Academic press (Molecular), and many other standard textbooks, all incorporated herein by reference.

The stringent conditions may include, for example, an overnight incubation at 42°C of a DNA-immobilized filter and a DNA probe in a solution containing 50% formamide, 5× SSC (750 mM sodium chloride, 75 mM sodium citrate), 50 mM sodium phosphate (pH 7.6), 5× Denhardt's solution, 10% dextran sulfate, and 20 µg/L denatured salmon sperm DNA, followed by washing the filter for example in a 0.2× SSC solution at approximately 65°C. Less stringent conditions may also be employed. Changes in stringency may be accomplished through the manipulation of formamide concentration (lower percentages of formamide result in lower stringency), salt concentrations, or temperature. For example, low stringent conditions include an overnight incubation at 37°C in a solution containing 6× SSCE (20× SSCE: 3 mol/L sodium chloride, 0.2 mol/L sodium dihydrogen phosphate, 0.02 mol/L EDTA, pH 7.4), 0.5% SDS, 30% formamide, and 100 µg/L denatured salmon sperm DNA, followed by washing in a 1× SSC solution containing 0.1% SDS at 50°C. To achieve even lower stringency, washes performed following hybridization may be done at higher salt concentrations (e.g., 5× SSC) in the above-mentioned low stringent conditions.

Variations in the above conditions may be accomplished through the inclusion or substitution of blocking reagents used to suppress background in hybridization experiments. The inclusion of blocking reagents may require modification of the hybridization conditions for compatibility.

The DNA capable of hybridizing under stringent conditions as described above may have a nucleotide sequence with at least 80%, preferably at least 90%, more preferably at least 95%, still more preferably at least 98%, particularly preferably at least 99% sequence identity to the nucleotide sequence of SEQ ID NO:1 as calculated using a program such as BLAST or FASTA with the parameters mentioned above.

Whether the DNA which hybridizes to the above-mentioned DNA under stringent conditions codes for a protein having a predetermined enzyme activity may be determined by conventional techniques, such as by expressing a target protein in a transformant produced by introducing a gene coding for the target protein into *Escherichia coli* or other host organisms, and determining the presence or absence of the function of the target protein by the corresponding activity measuring method.

Conventional techniques may be employed to identify the amino acid sequence and nucleotide sequence of the protein. For example, total RNA is extracted from a growing plant, the mRNA is optionally purified, and a cDNA is synthesized by a reverse transcription reaction. Subsequently, degenerate primers are designed based on the amino acid sequence of a known protein corresponding to the target protein, a DNA fragment is partially amplified by RT-PCR, and the sequence is partially identified. Then, the full-length nucleotide sequence and amino acid sequence is identified, e.g., by the RACE method. The RACE method (rapid amplification of cDNA ends method) refers to a method in which, when the nucleotide sequence of a cDNA is partially known, PCR is performed based on the nucleotide sequence data of such a known region to clone an unknown region extending to the cDNA terminal. This method is capable of cloning full-length cDNA by PCR without preparing a cDNA library.

The degenerate primers may each preferably be prepared from a plant-derived sequence having a highly similar sequence part to the target protein.

If the nucleotide sequence coding for the protein is known, the full-length nucleotide sequence and amino acid sequence can be identified by designing a primer containing a start codon and a primer containing a stop codon using the known nucleotide sequence, followed by performing RT-PCR using a synthesized cDNA as a template.

### (Transgenic plant preparation step)

The following describes the step (transgenic plant preparation step) of introducing a gene coding for a cis-prenyltransferase (CPT) family protein derived from a rubber-producing plant A into a rubber-producing plant B to prepare a transgenic plant C.

Once the rubber-producing plants to be used as the rubber-producing plants A and B are determined in view of the molecular weights of the natural rubbers produced by rubber-producing plants according to the above-mentioned technique, a person skilled in the art can carry out the transgenic plant preparation step.

After the determination of the rubber-producing plants A and B, if the CPT gene derived from the rubber-producing plant A is an unknown CPT gene, one may identify the DNA coding for a CPT family protein and then isolate the CPT gene by the above-mentioned techniques.

Then, the CPT gene derived from the rubber-producing plant A may be introduced into the rubber-producing plant B to prepare a transgenic plant C by a known method.

The following briefly describes a method for preparing the transgenic plant, although such a transgenic plant may be prepared by conventionally known methods.

A vector containing the nucleotide sequence of a CPT gene (CPT-containing vector) may be used for the transformation. When such a vector is introduced into a rubber-producing plant to cause transformation, the CPT gene in the vector will be expressed to allow the plant to produce a natural rubber with an increased molecular weight. In other words, a transgenic plant transformed to express a CPT family protein having a high ability to elongate the isoprene chain can be produced by introducing a CPT-containing vector into a rubber-producing plant. Then, the CPT family protein having a high ability to elongate the isoprene chain is expressed in the transgenic plant to allow the plant to produce a natural rubber with an increased molecular weight.

The CPT-containing vector may be prepared by inserting the nucleotide sequence of a CPT gene into a vector generally known as a plant transformation vector by conventional methods. Examples of vectors that can be used to prepare the CPT-containing vector include pBI vectors, binary vectors such as pGA482, pGAH, and pBIG, intermediate plasmids such as pLGV23Neo, pNCAT, and pMON200, and pH35GS containing GATEWAY cassette.

The CPT-containing vector may contain additional nucleotide sequences, provided that the nucleotide sequence of a CPT gene is present in the vector. Usually, the vector contains vector-derived sequences in addition to these nucleotide sequences and further contains a promoter sequence, a restriction enzyme recognition sequence, a spacer sequence, a marker gene sequence, a reporter gene sequence, or other sequences.

The CPT-containing vector is preferably a vector including a nucleotide sequence in which the CPT gene is functionally linked to a promoter.

The promoter may be any promoter that functions in plant cells. Examples of usable promoters include a cauliflower mosaic virus (CaMV) 35S promoter, a rice actin 1 promoter, a nopaline synthase gene promoter, a tobacco mosaic virus 35S promoter, a rice-derived actin gene promoter, and a ubiquitin promoter.

The promoter is preferably a promoter having a promoter activity that drives laticifer-specific gene expression.

Examples of the promoter having a promoter activity that drives laticifer-specific gene expression include a promoter of a gene coding for rubber elongation factor (REF), a promoter of a gene coding for small rubber particle protein (SRPP), a promoter of a gene coding for Hevein 2.1 (HEV2.1), and a promoter of a gene coding for MYC1 transcription factor (MYC1).

Herein, the term "promoter having a promoter activity that drives laticifer-specific gene expression" means that the promoter has activity to control gene expression to cause a desired gene to be expressed specifically in laticifers when the desired gene is functionally linked to the promoter and introduced into a plant. The term "laticifer-specific gene expression" means that the gene is considered to be expressed substantially exclusively in laticifers with no or little expression of the gene in sites other than laticifers in a plant. Also, the expression "a gene is functionally linked to a promoter" means that the gene sequence is linked downstream of the promoter so that the gene is controlled by the promoter.

Examples of the marker gene include drug-resistant genes such as a kanamycin-resistant gene, a hygromycin-resistant gene, and a bleomycin-resistant gene. The reporter gene is intended to be introduced to determine the expression site in a plant, and examples include a luciferase gene, a β-glucuronidase (GUS) gene, a green fluorescent protein (GFP), and a red fluorescent protein (RFP).

The CPT-containing vector may be introduced into a rubber-producing plant (including plant cells, such as calluses, cultured cells, spheroplasts, or protoplasts) by any method that can introduce DNA into plant cells. Examples include methods using Agrobacterium (JP S59-140885 A, JP S60-70080 A, and WO94/00977, all incorporated herein by reference), electroporation (JP S60-251887 A, incorporated herein by reference), and methods using particle guns (gene guns) (JP 2606856 B and JP 2517813 B, both incorporated herein by reference). Among these, it is preferred to use a method using Agrobacterium (Agrobacterium method) to introduce the CPT-containing vector into a plant to produce a transgenic plant (transgenic plant cells).

The above or other methods can be used to produce a transgenic plant (transgenic plant cells) in which a gene coding for a cis-prenyltransferase (CPT) family protein derived from a rubber-producing plant A is introduced into a rubber-producing plant B. The transgenic plant conceptually includes not only transgenic plant cells produced by the above methods, but also all of their progeny or clones and even progeny plants obtained by passaging the foregoing. Once obtaining transgenic plant cells into which a CPT-containing vector is introduced, progeny or clones can be produced from the transgenic plant cells by sexual or asexual reproduction, tissue culture, cell culture, cell fusion, or other techniques. Moreover, the transgenic plant cells, or their progeny or clones may be used to obtain reproductive materials (e.g., seeds, fruits, cuttings, stem tubers, root tubers, shoots, adventitious buds, adventitious embryos, calluses, protoplasts), which can then be used to produce the transgenic plant on a large scale.

Techniques to regenerate plants (transgenic plants) from transgenic plant cells are known; for example, Doi et al. disclose techniques for eucalyptus (JP H11-127025 A), Fujimura et al. disclose techniques for rice (Fujimura et al., (1995), Plant Tissue Culture Lett., vol. 2: p. 74-), Shillito et al. disclose techniques for corn (Shillito et al., (1989), Bio/Technology, vol. 7: p. 581-), Visser et al. disclose techniques for potato (Visser et al., (1989), Theor. Appl. Genet., vol. 78: p. 589-), and Akama et al. disclose techniques for *Arabidopsis thaliana* (Akama et al., (1992), Plant Cell Rep., vol. 12: p. 7-)(all the above documents are incorporated herein by reference). A person skilled in the art can regenerate plants from the transgenic plant cells in accordance with these documents.

Whether a target protein gene is expressed in regenerated plants may be determined by well-known methods. For example, Western blot analysis may be used to assess the expression of the target protein.

Seeds can be obtained from the transgenic plant, for example, as follows: the transgenic plant is rooted in an appropriate medium, transplanted to water-containing soil in a pot, and grown under proper cultivation conditions to finally produce seeds, which are then collected. Furthermore, plants can be grown from seeds, for example, as follows: the seeds obtained from the transgenic plant as described above are sown in water-containing soil and grown under proper cultivation conditions into plants.

### (Natural rubber production step)

The step (natural rubber production step) of producing a natural rubber using the transgenic plant C is described below.

In the transgenic plant C produced in the transgenic plant preparation step, the CPT family protein having a high ability to elongate the isoprene chain is expressed to allow the plant to produce a natural rubber with an increased molecular weight. Thus, a natural rubber with an increased molecular weight can be produced by growing the transgenic plant C so that the transgenic plant C can produce a natural rubber.

Specifically, a natural rubber with an increased molecular weight may be produced by culturing, for example, the transgenic plant cells produced in the transgenic plant preparation step, calluses obtained from the transgenic plant cells, or cells redifferentiated from the calluses in an appropriate medium, or growing, for example, transgenic plants regenerated from the transgenic plant cells, or plants grown from seeds obtained from the transgenic plants under proper cultivation conditions, so that the transgenic plant C can produce a natural rubber.

The natural rubber contained in the cells, culture medium, latex, or other material may be isolated and purified by known methods as needed.

The natural rubber produced by the transgenic plant may be obtained, for example, by harvesting latex from the transgenic plant and subjecting the latex to the solidification step below.

The harvesting of latex from the transgenic plant may be carried out by any method, including usual methods. For example, latex may be harvested by collecting the emulsion oozing out from cuts made in the trunk of the plant (tapping), or by collecting the emulsion oozing out from cuts made in roots or other parts of the transgenic plant, or by crushing the cut tissue followed by extraction with an organic solvent.

### <Solidification step>

The harvested latex may be subjected to a solidification step. The solidification may be carried out by any method, including, for example, by adding the latex to a solvent that does not dissolve natural rubber, such as ethanol, methanol, or acetone, or by adding an acid to the latex. The solidification step can be used to recover rubber (natural rubber) as solids from the latex. The obtained rubber (natural rubber) may be dried as needed before use.

Herein, the term "natural rubber" is a generic term for polymers composed of isoprene units (C₅H₈). Herein, the term "isoprenoid" refers to a compound having an isoprene unit (C₅H₈), and conceptually includes even natural rubber.

### (Method for producing rubber product)

The method for producing a rubber product of the first invention includes the steps of: kneading a natural rubber produced by the method for producing a natural rubber of the first invention with an additive to obtain a kneaded mixture ("kneading step"); forming a raw rubber product from the kneaded mixture ("raw rubber product forming step"); and vulcanizing the raw rubber product ("vulcanization step").

The rubber product may be any rubber product that can be produced from rubber, preferably natural rubber, and examples include pneumatic tires, rubber rollers, rubber fenders, gloves, and medical rubber tubes.

In the case where the rubber product is a pneumatic tire or, in other words, the method for producing a rubber product of the first invention is the method for producing a pneumatic tire of the first invention, the raw rubber product forming step corresponds to the step of forming a green tire from the kneaded mixture, and the vulcanization step corresponds to the step of vulcanizing the green tire. Thus, the method for producing a pneumatic tire of the first invention includes the steps of: kneading a natural rubber produced by the method for producing a natural rubber with an additive to obtain a kneaded mixture ("kneading step"); forming a green tire from the kneaded mixture ("green tire forming step"); and vulcanizing the green tire ("vulcanization step").

### <Kneading step>

In the kneading step, a natural rubber produced by the method for producing a natural rubber is kneaded with an additive to obtain a kneaded mixture.

Any additive may be used including those used in production of rubber products. For example, in the case where the rubber product is a pneumatic tire, examples of the additive include rubber components other than the natural rubber, reinforcing fillers such as carbon black, silica, calcium carbonate, alumina, clay, and talc, silane coupling agents, zinc oxide, stearic acid, processing aids, various antioxidants, softeners such as oils, waxes, vulcanizing agents such as sulfur, and vulcanization accelerators.

The kneading in the kneading step may be carried out using a rubber kneading machine such as an open roll mill, a Banbury mixer, or an internal mixer.

### <Raw rubber product forming step (green tire forming step in the case of tire)>

In the raw rubber product forming step, a raw rubber product (green tire in the case of tire) is formed from the kneaded mixture obtained in the kneading step.

The method of forming a raw rubber product is not particularly limited, and methods used to form raw rubber products may be used appropriately. For example, in the case where the rubber product is a pneumatic tire, the kneaded mixture obtained in the kneading step may be extruded according to the shape of a tire component and then assembled with other tire components in a usual manner on a tire building machine to form a green tire (unvulcanized tire).

### <Vulcanization step>

In the vulcanization step, the raw rubber product obtained in the raw rubber product forming step is vulcanized to produce a rubber product.

The method of vulcanizing the raw rubber product is not particularly limited, and methods used to vulcanize raw rubber products may be used appropriately. For example, in the case where the rubber product is a pneumatic tire, the green tire (unvulcanized tire) obtained in the raw rubber product forming step may be vulcanized by heating and pressing in a vulcanizer to produce a pneumatic tire.

### EXAMPLES

The present invention is specifically described with reference to examples, but the present invention is not limited to the examples.

### (Example 1)

### [Extraction of total RNA from Hevea latex]

Total RNA was extracted from the latex of *Hevea brasiliensis* by the hot phenol method. To 6 mL of the latex were added 6 mL of 100 mM sodium acetate buffer and 1 mL of a 10% SDS solution, and then 12 mL of water-saturated phenol pre-heated at 65°C. The mixture was incubated for five minutes at 65°C, agitated in a vortex mixer, and centrifuged at 7000 rpm for 10 minutes at room temperature. After the centrifugation, the supernatant was transferred to a new tube, 12 mL of a phenol:chloroform (1:1) solution was added, and they were agitated by shaking for two minutes. After the agitation, the resulting mixture was centrifuged again at 7000 rpm for 10 minutes at room temperature. Then, the supernatant was transferred to a new tube, 12 mL of a chloroform:isoamyl alcohol (24:1) solution was added, and they were agitated by shaking for two minutes. After the agitation, the resulting mixture was centrifuged again at 7000 rpm for 10 minutes at room temperature. Then, the supernatant was transferred to a new tube, 1.2 mL of a 3M sodium acetate solution and 13 mL of isopropanol were added, and they were agitated in a vortex mixer. The resulting mixture was incubated for 30 minutes at -20°C to precipitate total RNA. The incubated mixture was centrifuged at 15000 rpm for 10 minutes at 4°C, and the supernatant was removed to collect a precipitate of total RNA. The collected total RNA was washed twice with 70% ethanol and then dissolved in RNase-free water.

### [Synthesis of cDNA from total RNA]

cDNA was synthesized from the collected total RNA. The cDNA synthesis was carried out using a PrimeScript II 1st strand cDNA synthesis kit (Takara) in accordance with the manual.

### [Acquisition of CPT gene from cDNA]

The prepared 1st strand cDNA was used as a template to obtain a CPT gene. PCR was performed using a KOD-plus-Neo (Toyobo Co., Ltd.) in accordance with the manual. The PCR reaction involved 35 cycles with each cycle consisting of 10 seconds at 98°C, 30 seconds at 58°C, and 1 minute at 68°C.

The CPT gene was obtained using the following primers.
Primer 1: 5'-tttggatccgatggaattatacaacggtgagagg-3' (SEQ ID NO:3)
Primer 2: 5'-tttgcggccgcttattttaagtattccttatgtttctcc-3' (SEQ ID NO:4)

A CPT gene (HRT1) was produced as described above. The gene was sequenced to identify the full-length nucleotide sequence and amino acid sequence. The nucleotide sequence of HRT1 is given by SEQ ID NO:1. The amino acid sequence of HRT1 is given by SEQ ID NO:2.

### [Vector construction]

The obtained DNA fragment was subjected to dA addition and then inserted into a pGEM-T Easy vector using a pGEM-T Easy Vector System (Promega) to prepare pGEM-HRT1.

### [Transformation of Escherichia coli]

*Escherichia coli* DH5α was transformed with the prepared vector, and the transformant was cultured on LB agar medium containing ampicillin and X-gal. Then, *Escherichia coli* cells carrying the introduced target gene were selected by blue/white screening.

### [Plasmid extraction]

The *Escherichia coli* cells transformed with the plasmid containing the target gene were cultured overnight at 37°C in LB liquid medium. After the culture, the cells were collected, and the plasmid was collected using a FastGene Plasmid mini kit (Nippon Genetics Co., Ltd.).

It was confirmed by sequence analysis that there were no mutations in the nucleotide sequence of the collected gene inserted in the plasmid.

### [Preparation of vector for Lactuca sativa expression]

The pGEM-HRT1 acquired in the above [Vector construction] and a gateway vector for *Lactuca sativa* expression (pBI-pLsUbi-GW) were subjected to a LR reaction (insertion reaction, 25°C, two hours) to prepare a CPT-containing vector (pBI-pLsUbi-GW-HRT1). The pBI-pLsUbi-GW was obtained by substituting the 35S promoter of pBI-OX-GW with *Lactuca sativa* ubiquitin (LsUbi) promoter.

It was then confirmed by sequence analysis that there were no mutations in the nucleotide sequence of the gene inserted in the plasmid by the same technique as described for pGEM-HRT1.

### [Preparation of explant]

Seeds of *Lactuca sativa* (cultivar: Kaiser available from Takii & Co., Ltd.) were immersed in a five-fold dilution of a home bleaching agent for 15 minutes to sterilize the seed surface. Subsequently, the seeds were rinsed three times with sterilized water in a clean bench. Then, the surface-sterilized seeds were sown on a germination medium (MS medium + 2% sucrose + 0.3% Gelrite) at a rate of approximately 25 seeds per plant box, and then cultured at 25°C with a photoperiod of 16 hours light and 8 hours dark for five days.

### [Preparation of Agrobacterium]

A glycerol stock of Agrobacterium (*Agrobacterium tumefaciens*) into which the CPT-containing vector (pBI-pLsUbi-GW-HRT1) was introduced was inoculated into LB liquid medium and then shake-cultured in the liquid for about 24 hours. After completion of the culture, the Agrobacterium cells were collected by centrifugation (3000 rpm, five minutes), and the supernatant was discarded. The Agrobacterium cells were re-suspended with an infection medium (MS medium + 3% sucrose + 0.1 mM acetosyringone + 0.01 mM mercaptoethanol), followed by adjustment of the absorbance at 600 nm (OD 600) to about 0.8. The resulting bacterial culture was used for transformation.

### [Infection of Agrobacterium]

The infection medium (about 40 mL) in which the Agrobacterium cells were suspended was transferred to a sterilized dish. Then, the cotyledons produced in the above [Preparation of explant] were cut from the tip into 5 mm pieces, which were then directly dropped and introduced into the infection medium, followed by standing for about five minutes.

The cotyledon pieces left in the infection medium were then allowed to stand on a sterilized Kimtowel so that the Agrobacterium adhered to the cotyledon pieces was adsorbed to the Kimtowel.

The cotyledon pieces left on the Kimtowel were then plated on a co-culture medium (MS medium + 3% sucrose + 1 mg/L benzyladenine + 0.1 mg/L naphthaleneacetic acid + 0.3% Gelrite) with their adaxial surfaces being in contact with the medium. About 25 pieces were plated on a dish, which was then sealed with a surgical tape, and co-culture was conducted at 25°C in the dark for three days.

### [Callus induction and adventitious bud induction]

After the co-culture, the cotyledon pieces were transplanted onto a callus-inducing medium (MS medium + 3% sucrose + 0.1 mg/L benzyladenine (BA) + 0.1 mg/L naphthaleneacetic acid (NAA) + 100 mg/L kanamycin + 375 mg/L (1 tablet/L) Augmentin + 0.3% Gelrite) and cultured at 25°C under a lighting regime of 16 hours light and 8 hours dark for three weeks while changing the medium every week. In this way, calluses were induced while selection was performed.

Next, the cotyledon pieces with calluses were transplanted onto an adventitious bud-inducing medium (MS medium + 3% sucrose + 0.01 mg/L benzyladenine (BA) + 0.05 mg/L naphthaleneacetic acid (NAA) + 100 mg/L kanamycin + 375 mg/L (1 tablet/L) Augmentin + 0.3% Gelrite).

Adventitious buds were differentiated during the culture in the adventitious bud-inducing medium. Then, the adventitious buds were cut out from the calluses with a scalpel when the adventitious buds (true leaves) grew to a size of about 1 to 2 cm. The grown calluses were transplanted onto a rooting selection medium (MS medium + 3% sucrose + 100 mg/L kanamycin + 375 mg/L (1 tablet/L) Augmentin + 0.3% Gelrite). It should be noted that the culture in the adventitious bud-inducing medium was continued for about two months, but when the end of the grown calluses started to turn brown, the discolored part was cut out with a scalpel, followed by continuing the culture.

### [Rooting selection and acclimation]

Culture in the rooting selection medium was performed without changing the medium for about one month. Then, when a root grew to a certain length (a little less than 5 cm), the grown plant tissue was taken out of the medium, and the remaining medium was removed taking care not to damage the root.

Next, the grown plant tissues were transplanted onto a culture soil. The transplanted plants were placed in a tray and wrapped in a wrap to keep a sufficient humidity.

The plants were acclimated to low-humidity conditions over about ten days from the beginning of the acclimation by forming holes in the wrap or moving the lid every three or four days.

Thereafter, the fully acclimated plants were cultivated in a culture soil for eight weeks at 22°C with a 16-hour photoperiod.

### [Confirmation of transfection]

The DNA of the plants cultivated in the culture soil for eight weeks was extracted and analyzed by PCR to confirm transfection. Only the confirmed transfected individuals were continued to be cultivated.

### [Hydroponics/bolting]

The confirmed transfected individuals were transferred to hydroponics. The medium used in the hydroponics was a mixture of OAT house No. 1, No. 2, and No. 5 (OAT house fertilizer series, OAT Agrio Co., Ltd.) at a ratio of 30:30:1. In order to promote bolting, the cultivation was carried out at an air temperature range of 17 to 22°C without falling below 17°C.

### [Extraction of natural rubber]

The confirmed transfected individuals (*Lactuca sativa*) were cultivated for six months in hydroponics, and then latex was extracted from their stems and leaves. The extracted latex was immersed in ethanol. The formed solids were washed with ethanol to obtain a natural rubber.

### [Measurement of weight average molecular weight of natural rubber]

The molecular weight distribution of the natural rubber was measured under the below-mentioned conditions. Table 1 shows the results.

### Pre-treatment for measurement

(1) Removal of ethanol and then evaporation to dryness (six hours to overnight)
(2) Weighing, addition of tetrahydrofuran to a concentration of 0.1%, and then standing (overnight)
(3) Filtration and then measurement

The weight average molecular weight (Mw) was measured by gel permeation chromatography (GPC) under the following conditions (1) to (7).
(1) Apparatus: HLC-8320GPC available from Tosoh Corporation
(2) Separation column: TSKgel SM HZ-H available from Tosoh Corporation
(3) Measurement temperature: 40°C
(4) Carrier: tetrahydrofuran
(5) Flow rate: 0.6 mL/min
(6) Detector: differential refractometer, UV
(7) Molecular weight standards: polystyrene standards

### (Comparative Example 1)

An experiment was performed as in Example 1, except that pBI-pLsUbi-GW (CPT-free vector) was used instead of the CPT-containing vector (pBI-pLsUbi-GW-HRT1).

**[Table 1]**

| | Comparative Example 1 | Example 1 |
|---|---|---|
| Weight average molecular weight | 1.5 × 10⁶ | 1.8 × 10⁶ |

The weight average molecular weights indicated in Table 1 are each the average of four weight average molecular weights measured in each example.

The results in Table 1 demonstrated that a natural rubber with an increased molecular weight (weight average molecular weight) can be produced by a method for producing a natural rubber which includes the steps of: introducing a gene coding for a cis-prenyltransferase (CPT) family protein derived from a rubber-producing plant A (*Hevea brasiliensis*) into a rubber-producing plant B (*Lactuca sativa*) to prepare a transgenic plant C; and producing a natural rubber using the transgenic plant C, wherein the rubber-producing plant A produces a natural rubber having a higher molecular weight than the natural rubber produced by the rubber-producing plant B not transfected with the gene.

It was also proved that a natural rubber with an increased molecular weight (weight average molecular weight) can be produced by a transgenic plant produced by introducing a gene coding for a cis-prenyltransferase (CPT) family protein derived from a rubber-producing plant A (*Hevea brasiliensis*) into a rubber-producing plant B (*Lactuca sativa*), wherein the rubber-producing plant A produces a natural rubber having a higher molecular weight than the natural rubber produced by the rubber-producing plant B not transfected with the gene.

### (SEQUENCE LISTING FREE TEXT)

SEQ ID NO:1: Nucleotide sequence of gene coding for HRT1 from *Hevea brasiliensis*
SEQ ID NO:2: Amino acid sequence of HRT1 from *Hevea brasiliensis*
SEQ ID NO:3: Primer 1
SEQ ID NO:4: Primer 2

To provide methods for producing a natural rubber which enable the production of a natural rubber with an increased molecular weight, and transgenic plants which are able to produce a natural rubber with an increased molecular weight. Included is a method for producing a natural rubber, the method including the steps of: introducing a gene coding for a cis-prenyltransferase (CPT) family protein derived from a rubber-producing plant A into a rubber-producing plant B to prepare a transgenic plant C; and producing a natural rubber using the transgenic plant C, the rubber-producing plant A producing a natural rubber having a higher molecular weight than a natural rubber produced by the rubber-producing plant B not transfected with the gene.

## Claims

1. A method for producing a natural rubber, the method comprising the steps of:
introducing a gene coding for a cis-prenyltransferase (CPT) family protein derived from a rubber-producing plant A into a rubber-producing plant B to prepare a transgenic plant C; and
producing a natural rubber using the transgenic plant C,
the rubber-producing plant A producing a natural rubber having a higher molecular weight than a natural rubber produced by the rubber-producing plant B not transfected with the gene.

2. The method for producing a natural rubber according to claim 1,
wherein the gene coding for a cis-prenyltransferase (CPT) family protein is a gene coding for a cis-prenyltransferase (CPT) family protein derived from a plant of the genus *Hevea, Ficus, Taraxacum,* or *Parthenium.*

3. The method for producing a natural rubber according to claim 1,
wherein the gene coding for a cis-prenyltransferase (CPT) family protein is a gene coding for a cis-prenyltransferase (CPT) family protein derived from *Hevea brasiliensis, Taraxacum kok-saghyz,* or *Parthenium argentatum.*

4. A method for producing a pneumatic tire, the method comprising the steps of:
kneading a natural rubber produced by the method for producing a natural rubber according to any one of claims 1 to 3 with an additive to obtain a kneaded mixture;
forming a green tire from the kneaded mixture; and
vulcanizing the green tire.

5. A method for producing a rubber product, the method comprising the steps of:
kneading a natural rubber produced by the method for producing a natural rubber according to any one of claims 1 to 3 with an additive to obtain a kneaded mixture;
forming a raw rubber product from the kneaded mixture; and
vulcanizing the raw rubber product.

6. A transgenic plant, produced by introducing a gene coding for a cis-prenyltransferase (CPT) family protein derived from a rubber-producing plant A into a rubber-producing plant B,
the rubber-producing plant A producing a natural rubber having a higher molecular weight than a natural rubber produced by the rubber-producing plant B not transfected with the gene.


SEQUENCE LISTING

<110>  SUMITOMO RUBBER INDUSTRIES, LTD.

<120>  METHOD FOR PRODUCING NATURAL RUBBER, TRANSGENIC PLANT, METHOD FOR
PRODUCING PNEUMATIC TIRE, AND METHOD FOR PRODUCING RUBBER PRODUCT

<130>  SR2732

<150>  JP 2019-103713

<151>  2019-06-03

<160>  4

<170>  PatentIn version 3.5

<210>  1
<211>  873
<212>  DNA
<213>  Hevea brasiliensis

<400>  1
atggaattat acaacggtga gaggccaagt gtgttcagac ttttagggaa gtatatgaga      60

aaagggttat atagcatcct aacccagggt cccatcccta ctcatattgc cttcatattg     120

gatggaaaca ggaggtttgc taagaagcat aaactgccag aaggaggtgg tcataaggct     180

ggatttttag ctcttctgaa cgtactaact tattgctatg agttaggagt gaaatatgcg     240

actatctatg cctttagcat cgataatttt cgaaggaaac ctcatgaggt tcagtacgta     300

atggatctaa tgctggagaa gattgaaggg atgatcatgg aagaaagtat catcaatgca     360

tatgatattt gcgtacgttt tgtgggtaac ctgaagcttt taagtgagcc agtcaagacc     420

gcagcagata agattatgag ggctactgcc aacaattcca aatgtgtgct tctcattgct     480

gtatgctata cttcaactga tgagatcgtg catgctgttg aagaatcctc tgaattgaac     540

tccaatgaag tttgtaacaa tcaagaattg gaggaggcaa atgcaactgg aagcggtact     600

gtgattcaaa ttgagaacat ggagtcgtat tctggaataa aacttgtaga ccttgagaaa     660

aacacctaca taaatcctta tcctgatgtt ctgattcgaa cttctgggga gacccgtctg     720

agcaactact tactttggca gactactaat tgcatactgt attctcctca tgcactgtgg     780

ccagagattg gtcttcgaca cgtggtgtgg gcagtaatta acttccaacg tcattattct     840

tacttggaga aacataagga atacttaaaa taa                                  873


<210>  2
<211>  290
<212>  PRT
<213>  Hevea brasiliensis

<400>  2
Met Glu Leu Tyr Asn Gly Glu Arg Pro Ser Val Phe Arg Leu Leu Gly
1               5                   10                  15

Lys Tyr Met Arg Lys Gly Leu Tyr Ser Ile Leu Thr Gln Gly Pro Ile
            20                  25                  30

Pro Thr His Ile Ala Phe Ile Leu Asp Gly Asn Arg Arg Phe Ala Lys
        35                  40                  45

Lys His Lys Leu Pro Glu Gly Gly Gly His Lys Ala Gly Phe Leu Ala
    50                  55                  60

Leu Leu Asn Val Leu Thr Tyr Cys Tyr Glu Leu Gly Val Lys Tyr Ala
65                  70                  75                  80

Thr Ile Tyr Ala Phe Ser Ile Asp Asn Phe Arg Arg Lys Pro His Glu
                85                  90                  95

Val Gln Tyr Val Met Asp Leu Met Leu Glu Lys Ile Glu Gly Met Ile
            100                 105                 110

Met Glu Glu Ser Ile Ile Asn Ala Tyr Asp Ile Cys Val Arg Phe Val
        115                 120                 125

Gly Asn Leu Lys Leu Leu Ser Glu Pro Val Lys Thr Ala Ala Asp Lys
    130                 135                 140

Ile Met Arg Ala Thr Ala Asn Asn Ser Lys Cys Val Leu Leu Ile Ala
145                 150                 155                 160

Val Cys Tyr Thr Ser Thr Asp Glu Ile Val His Ala Val Glu Glu Ser
                165                 170                 175

Ser Glu Leu Asn Ser Asn Glu Val Cys Asn Asn Gln Glu Leu Glu Glu
            180                 185                 190

Ala Asn Ala Thr Gly Ser Gly Thr Val Ile Gln Ile Glu Asn Met Glu
        195                 200                 205

Ser Tyr Ser Gly Ile Lys Leu Val Asp Leu Glu Lys Asn Thr Tyr Ile
    210                 215                 220

Asn Pro Tyr Pro Asp Val Leu Ile Arg Thr Ser Gly Glu Thr Arg Leu
225                 230                 235                 240

Ser Asn Tyr Leu Leu Trp Gln Thr Thr Asn Cys Ile Leu Tyr Ser Pro
                245                 250                 255

His Ala Leu Trp Pro Glu Ile Gly Leu Arg His Val Val Trp Ala Val
            260                 265                 270

Ile Asn Phe Gln Arg His Tyr Ser Tyr Leu Glu Lys His Lys Glu Tyr
        275                 280                 285

Leu Lys
    290

<210>  3
<211>  34
<212>  DNA
<213>  Artificial Sequence

<220>
<223>  PRIMER-1

<400>  3
tttggatccg atggaattat acaacggtga gagg                                    34

<210>  4
<211>  39
<212>  DNA
<213>  Artificial Sequence

<220>
<223>  PRIMER-2

<400>  4
tttgcggccg cttattttaa gtattcctta tgtttctcc                               39